# EUROPÄISCHE PATENTSCHRIFT

(11) **EP 0 291 021 B1**
(45) Veröffentlichungstag und Bekanntmachung des Hinweises auf die Patenterteilung: **10.02.1993**
(21) Anmeldenummer: 88107519.6
(22) Anmeldetag: 10.05.1988
(51) Int. Cl.: C12N 15/62, C12P 21/00, C12N 9/36

(54) **Für ein lytisch wirksames, chimäres Protein kodierende rekombinante DNA und ihre Verwendung**
Recombinant DNA encoding a chimaeric protein with a lytic activity, and its application
ADN recombinant, codant pour une protéine chimérique ayant une activité lytique et son application

(30) Priorität: 12.05.1987 DE 3715840
(43) Veröffentlichungstag der Anmeldung: 17.11.1988
(73) Patentinhaber: Lubitz, Werner, Dr., 1030 Wien (AT)
(72) Erfinder: Lubitz, Werner, Dr. rer. nat., D-8000 München 2 (DE); Harkness, Robin Edmond, Dr. rer. nat., D-7400 Tübingen (DE)
(74) Vertreter: Huber, Bernhard, Dipl.-Chem.

(56) Entgegenhaltungen:
- FEMS MICROBIOLOGY LETTERS, Band 48, 1987, Seiten 19-24, Elsevier, Amsterdam, NL; R.E. HARKNESS et al.: "Construction and properties of a chimeric bacteriophage lysis gene"
- GENE, Band 37, 1985, Seiten 171-179, Elsevier Science Publishers, Amsterdam, NL; B. BERKHOUT et al.: "Translational interference at overlapping reading frames in prokaryotic messenger RNA"
- JOURNAL OF GENERAL VIROLOGY, Band 66, 1985, Seiten 1209-1213, DGM, Colchester, GB; U. BLÄSI et al.: "Influence of C-terminal modifications of phiX174 lysis gene E on its lysis-inducing properties"

## Beschreibung

Die Erfindung betrifft rekombinante DNA, Verfahren zu ihrer Herstellung und ihre Verwendung zur Gewinnung von eukaryontischen oder prokaryontischen Stoffwechselprodukten oder gentechnologisch hergestellten Proteinen.

Die Bedeutung der gentechnologischen Herstellung von Stoffwechselprodukten und Proteinen ist in den letzten Jahren stetig angestiegen. So gelingt es auf diese Weise bisher nur sehr umständlich und in kleinen Mengen isolierbare und damit teure Substanzen, wie z. B. Interferone, Interleukine, Insulin etc., im großtechnischen Maßstab herzustellen, und damit ihre Anwendung, z. B. in Arzneimitteln, möglich zu machen. Nach der Herstellung solcher Substanzen in der verwendeten Zellinie ist jedoch auch ihre Isolierung teilweise sehr aufwendig, da die Zellen zuerst zerstört werden müssen, um die gebildete Substanz freizusetzen, dann aber eine Aufreinigung aus dem Medium erforderlich ist, bei der alle Zellbruchstücke und -bestandteile abgetrennt werden müssen. Diese Zerstörung der Zellen, Lysis genannt, kann auf mehrere Arten durchgeführt werden. So ist es möglich, ein lytisch wirksames Enzym, wie z. B. Lysozym, einzusetzen oder aber die Zellen durch das Anlegen eines osmotischen Drucks oder mit Hilfe von Ultraschall oder anderen biochemischen oder physikalischen Methoden aufzuschließen.

Günstiger wäre jedoch eine spezifische Lysis bestimmter Membranen, oder aber die Ausschleusung der Substanzen durch teilweise permeabel gemachte Membranen, wobei sogar eine kontinuierliche Fortsetzung der Produktion von Substanzen ermöglicht würde.

Für die Gewinnung von gentechnologisch hergestellten Produkten durch Ausschleusung oder aber Lysis der Zellen wäre es daher von großem Vorteil, über möglichst stark lytisch wirksame Proteine zu verfügen, die zusätzlich eine Spezifität für bestimmte Membranen aufweisen oder aber eine Ausschleusung dadurch möglich machen, daß sie spezifische Zellmembranen für das entsprechende Produkt permeabel machen. Ebenso wäre es günstig, solche lysierenden Proteine nicht von außen der Zelle zuführen zu müssen, sondern die Proteine bereits in der Zelle zu einem gewünschten Zeitpunkt bilden zu können. Der Erfindung lag daher die Aufgabe zugrunde, eine für ein Protein, das diesen Forderungen gerecht wird, kodierende rekombinante DNA bereitzustellen, deren Verwendung die Gewinnung von gentechnologisch hergestellten Produkten erleichtert.

Gelöst wird diese Aufgabe durch rekombinante DNA, die dadurch gekennzeichnet ist, daß sie für die N-terminale, membrandurchdringende Domäne des E-Proteins des Phagen φX174 kodierende DNA-Sequenzen und für die C-terminale, membrandurchdringende Domäne des L-Proteins des Phagen MS2 kodierende DNA-Sequenzen enthält und die DNA-Sequenzen der beiden Phagen durch eine für eine hydrophile flexible Aminosäuresequenz kodierende DNA-Sequenz verbunden sind.

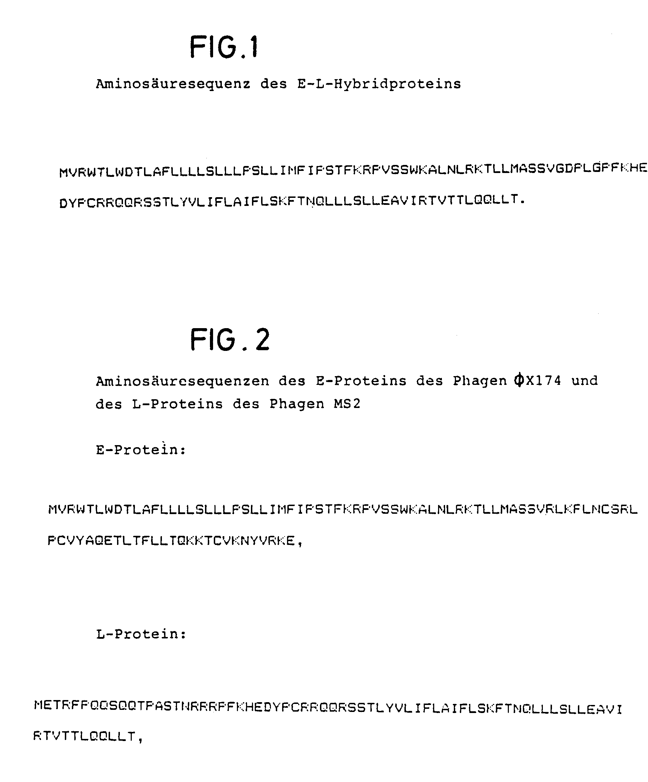

Der einzelsträngige DNA-Phage φX174 und der einzelsträngigen RNA-Phage MS2 enthalten jeweils ein phagenkodiertes, lytisches Protein, nämlich das E-Protein (φX174) und das L-Protein (MS2). Diese beiden lytischen Proteine sind relativ klein und in ihrer gesamten Aminosäuresequenz bekannt. Diese Aminosäuresequenzen sind in Fig. 2 gezeigt. Es ist daraus ersichtlich, daß das E-Protein 91 Aminosäuren und das L-Protein 75 Aminosäuren lang ist. Beide lytisch wirksamen Proteine enthalten jedoch keine enzymatische Aktivität. Es ist daher anzunehmen, daß sie die Lysis von Membranen durch Zerstörung der Membranstruktur in Verbindung mit autolytischen Prozessen bewirken. Die lytische Aktivität wurde für das E-Protein von Bläsi und Lubitz, J. Gen. Virol. 66:1209-1213 (1985) und Schüller A. et al, Nucl. Acids Res. 13:4143-4153 (1985), der membrandurchdringenden N-terminalen Region zugeschrieben. Zusätzlich scheint für die Funktionalität die Anwesenheit einer oligomerisierenden Struktur im C-terminalen Bereich des Proteins nötig zu sein, wie von Maratea et al, Gene 40:39-64 (1985) und Buckley und Hayashi, Mol. Gen. Genet. 204:120-125 (1986) festgestellt wurde. Aus durch Computerberechnung erstellte Proteinstrukturvoraussagen wird für das L-Protein eine entgegengesetzte funktionelle Orientierung angenommen. Demnach ist die Domäne, welche die lytische Aktivität bewirkt, auf den C-terminalen Bereich des L-Proteins beschränkt (Berkhout et al, Gene 37:171-179 (1985)).

Die erfindungsgemäße rekombinante DNA ermöglicht es, ein Fusionsprotein aus dem N-terminalen, alleine nicht lytisch wirksamen Bereich des Proteins E und des ebenfalls alleine nicht lytisch wirksamen C-terminalen, membrandurchdringenden Teils des L-Proteins herzustellen, das als Fusionsprotein, wobei die beiden Phagen-DNA-Sequenzen durch eine hydrophile flexible Aminosäuresequenz verbunden sind, eine bedeutend stärkere lytische Aktivität aufweist, als die Ausgangsproteine selbst.

Bevorzugte Ausführungsformen der Erfindung sind in den Unteransprüchen gekennzeichnet. Eine bevorzugte rekombinante DNA enthält die DNA-Sequenz, welche für die Aminosäuren 1 bis 54 des Proteins E des Phagen φX174 kodiert und die DNA-Sequenz, welche für die Aminosäuren 21 bis 75 des Proteins L des Phagen MS2 kodiert, wobei diese DNA-Sequenzen durch eine weitere DNA-Sequenz verbunden sind, welche für fünf Linkeraminosäuren kodiert.

Besonders bevorzugt enthält die rekombinante DNA eine DNA-Sequenz, welche für eine Aminosäurekette kodiert, wie sie in Fig. 1 dargestellt ist.

Eine solche rekombinante DNA kann eingebaut in einen Vektor vorliegen. Als Vektor wird hierbei bevorzugt ein Plasmid oder ein Phagengenom verwendet. Bevorzugt befindet sich vor den Phagen-DNA-Sequenzen ein Promotor, der die Expression der rekombinanten DNA steuert. Als Promotoren sind hierbei prokaryontische oder eukaryontische Promotoren, insbesondere regulierbare Promotoren, geeignet. Bevorzugt sind der Lamda-Promotor, der lac-Promotor oder der Ga110-Promotor.

Ein weiterer Gegenstand der Erfindung ist eine rekombinante DNA, die vor den Phagensequenzen zusätzlich eine für eine Signalsequenz kodierende DNA-Sequenz enthält. Durch die Anwesenheit einer Signalsequenz im exprimierten Protein ist es möglich, diesen eine Spezifität für bestimmte Membranen zu übertragen.

Ein weiterer Gegenstand der Erfindung ist das Plasmid pRM17, DSM 4092P, das dadurch gekennzeichnet ist, daß es rekombinante DNA enthält, welche für eine Aminosäuresequenz codiert, wie sie in Fig. 1 dargestellt ist.

Das erfindungsgemäße Verfahren zur Herstellung der erfindungsgemäßen rekombinanten DNA ist dadurch gekennzeichnet, daß man nach an sich bekannten Methoden die entsprechenden DNA-Sequenzen aus doppelsträngigen DNA-Form des Phagen φX174 und der DNA-Kopie der Phagen MS2 RNA isoliert und miteinander in der gewünschten Reihenfolge ligiert.

Die auf diese Weise hergestellte rekombinante DNA kann dann in einen Vektor insertiert werden, wobei als Vektor bevorzugt ein Plasmid oder Phagengenom verwendet wird. Weiterhin ist es bevorzugt, in den Vektor, vor die Phagen-DNA-Sequenz, zusätzlich einen eukaryontischen oder prokaryontischen Promotor zu insertieren. Besonders bevorzugt verwendet man einen Vektor, der bereits einen Promotor enthält, unter dessen Kontrolle ein Fremdgen exprimiert werden kann.

Bevorzugt wird bei der Herstellung einer rekombinanten DNA vor die Phagen-DNA-Sequenz zusätzlich eine für eine Signalsequenz kodierende DNA-Sequenz insertiert.

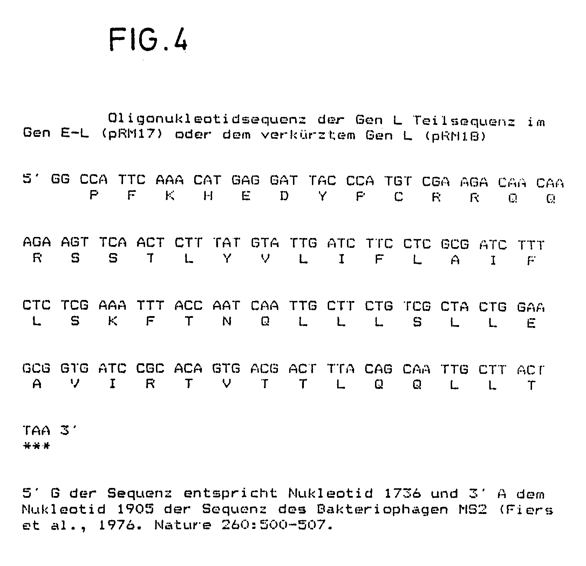

Das Plasmid pRM17, DSM 4092P, kann dadurch hergestellt werden, daß man das Oligonucleotid gemäß Fig. 4 in das mit XbaI/ HindIII geschnittene Plasmid pSU729 (Nucl. Acids Res. 13, 4145-4153; 1985) insertiert, wobei die XbaI-Stelle des Plasmids pSU729 vorher mit DNA-Polymerase I (Klenow-Fragment) aufgefüllt wird.

Ein weiterer Gegenstand der Erfindung ist die Verwendung einer rekombinanten DNA oder des Plasmids pRM17, DSM 4092P, zur Gewinnung von eukaryontischen oder prokaryontischen Stoffwechselprodukten oder gentechnologisch hergestellten Proteinen durch partielle oder vollständige Lysis von Membranen oder der Zellwand der zur Expression verwendeten Zellen oder Mikroorganismen durch Expression des durch die rekombinante DNA kodierten lytischen Proteins.

Bevorzugt wird hierbei die Expression des lytischen Proteins unter Kontrolle eines regulierbaren Promotors zu einem gewünschten Zeitpunkt induziert. Dies ermöglicht es, Zellen erst bis zu einer bestimmten Wachstumsphase heranwachsen zu lassen und dadurch die Produktion des gewünschten Stoffwechselprodukts oder des gentechnologisch hergestellten Proteins in möglichst effektiver Weise durchzuführen, bevor durch Induktion der Expression des lytischen Proteins die Zellwand teilweise oder vollständig lysiert wird, und dadurch die gewünschten Produkte ins Medium gelangen, woraus sie problemlos isoliert werden können.

Als Promotoren sind im Prinzip alle induzierbaren (regulierbaren Promotoren geeignet. Beispiele hierfür sind der Promotor λ_{PL} bzw. λ_{PR} mit CI 857 als Repressor (Gene 5 (1979) 59) und die Promotor-Operator-Region Lac PO mit Lac I^{q} oder Lac I^{q1} als Repressor (Mol.Gen. Genet. 185, (1982) 493-497).

Weiterhin kann durch das Vorhandensein einer Signalsequenz im lytischen Protein die spezifische Lysis bestimmter Membranen erreicht werden. Insbesondere kann hierdurch auch die Lysis von Organellenmembranen bewirkt werden, wie z. B. von Lysosomen, Mitochondrien oder Chloroplasten. Es werden hierbei eine an sich bekannte Signalsequenz vor den N-terminalen Teil des lytischen Fusionsprotein angehängt, die spezifisch für eine bestimmte Membran ist und eine Inkorporation des Fusionsproteins in eben diese Membran bewirkt.

Durch nur teilweise Lysis bestimmter spezifischer Membranen wird die Ausschleusung der Stoffwechselprodukte oder Proteine erreicht, wodurch eine kontinuierlich fortlaufende Produktion ermöglicht wird, ohne daß das Abtöten der produzierenden Zellen nötig ist. Die hergestellten Stoffwechselprodukte können dann einfach aus dem Wachstumsmedium isoliert werden, eine umständliche Abtrennung von Zellbestandteilen ist nicht mehr nötig.

Folgende Abbildungen erläutern die Erfindung weiter:
- Fig. 1:: Aminosäuresequenz des E-L-Hybridproteins
- Fig. 2:: Aminosäuresequenz des E-Proteins des Phagen φX174 und des L-Proteins des Phagen MS2
- Fig. 3:: Wachstum des E.coli-Stamm K12, PC2479, DSM 4089, unter Expression der Plasmide pSB12 (▲ - ▲., φX174 Gen E), pMS 1.17 (● - ● ; MS2-Gen L), pRM17, (■ - ■, rekombinantes E-L-Gen), pSU 730-1 (□ - □ ; E-Untereinheit des rekombinanten E-L-Gens) und pRM18 (○ - ○ ; L-Untereinheit des rekombinanten E-L-Gens)
- Fig. 4:: Oligonukleotidsequenz der Gen-L-Teilsequenz im Gen E-L (pRM17) oder dem verkürzten Gen L (pRM18)

Die folgenden Beispiele sollen die Erfindung weiter erläutern:

### Beispiel 1

### Herstellung von pRM 17

Das chimäre Gen E-L des Plasmids pRM 17 erhält man durch Insertion der Oligonukleotidsequenz (Gen-L-Teilsequenz, Fig. 4), die den Aminosäurecodons 21-75 des Gen L des Phagen MS2 entspricht (Beremand, M.N. & T. Blumenthal, 1979. Cell 18: 257-266), in die aufgefüllte XbaI-Stelle des Plasmids pSU729. 5′G der Sequenz entspricht Nucleotid 1736 und 3′A dem Nucleotid 1905 der Sequenz des Bakteriophagen MS2 (Fiers et al., 1976, Nature 260, 500-507). Dieses Plasmid (pSU729) wurde durch Insertion der PhiX174 Teilsequenz nt 447-729 in die EcoRi/SmaI-Stellen des Plasmids pSU1 konstruiert (Schüller et al., 1985, Nucl. Acids Res. 13, 4143-4153). Die Orientierung der Oligonukleotidsequenz in pRM17 ist 5′ - 3′ entsprechend der Orientierung der PhiX174 Gen E Teilsequenz (Codon 1-54), die auf diesem Plasmid enthalten ist. Die Polylinkersequenz zwischen der Gen E Teilsequenz und Gen L Teilsequenz entspricht

5' GGG GAT CCT CTA G 3'
G D P L (G)

### Beispiel 2

### Herstellung von pRM18

Durch Insertion der Oligonukleotidsequenz (Gen-L-Teilsequenz, Fig. 4) in die aufgefüllte BamHI-Stelle des Plasmids pPLcAT10 (Stanssens et al., 1985, Gene 36, 211-223) erhält man Plasmid pRM18. Die Orientierung des Oligonukleotids in pRM18 ist 5′ - 3′ entsprechend der 5′-seitig zur BamHI-Stelle auf dem Plasmid enthaltenen Ribosomenbindungsstelle und des ATG Startcodons. Plasmidsequenzen, die zum verkürzten Protein-L beitragen und vor der Oligonukleotidsequenz des Gen-L-Teilfragements liegen, sind

5' ATG GAT C 3'
M D (R)

### Beispiel 3

### Lysis von E. coli-Zellen durch plasmidkodiertes E-, L-und E-L-Protein.

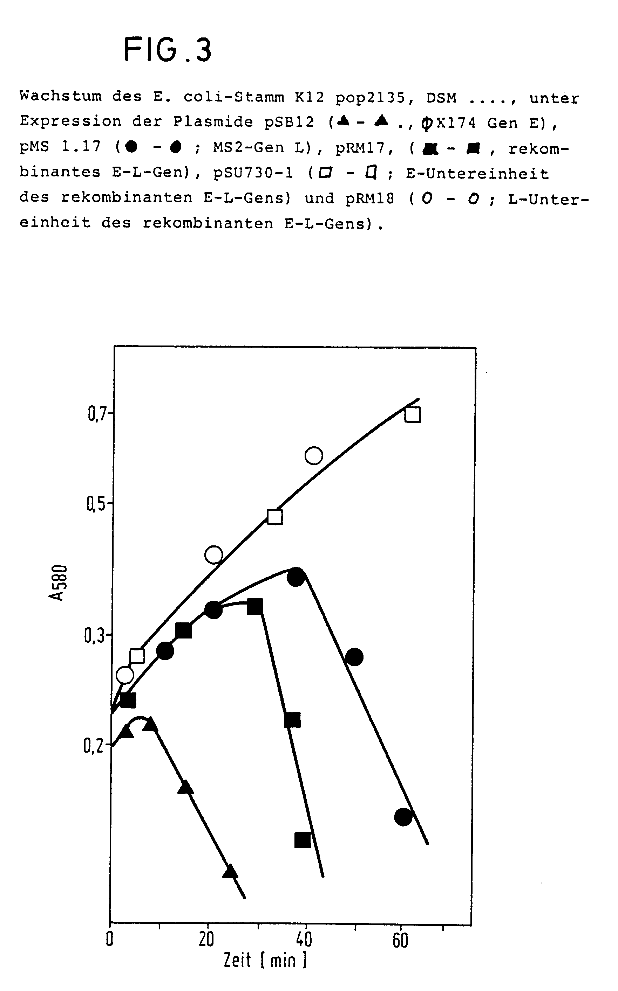

Die Lysis von E. coli Zellen durch die lytisch wirksamen Proteine E, L und das aus erfindungsgemäßer rekombinanter DNA hergestellte E-L-Protein wurde im E. coli-Stamm K12 PC2479, DSM 4089, untersucht. Hierbei wurden außer dem erfindungsgemäßen Plasmid pRM17, DSM 4092P, das Plasmid pSB12, DSM 4091P, welches das φX174 Gen E enthält (J.Gen.Microbiol. 131 (1985) 1107-1114), das Plasmid pMS1.17, DSM 4094P, welches das MS2-Gen L enthält (Nature 305 (1983) 741-743), das Plasmid pSU730-1, DSM 4095P, welches die E-Untereinheit des erfindungsgemäßen rekombinanten Gens enthält (Nucl. Acids Res. 13 (1985) 4143-4153), und das Plasmid pRM18, DSM 4093P, welches die L-DNA-Untereinheit des rekombinanten E-L-Gens enthält, verwendet. Die Expression der plasmidkodierten Gene wurde dabei unter Kontrolle des Lamda-P_{L}-Promotors gesteuert (CI 857 als Repressor, vgl Gene 5 (1979) 59). Um die Expression der Plasmide zu induzieren, wurde die Wachstumstemperatur der exponentiell wachsenden Kulturen von 28°C auf 42°C erhöht. Das Wachstum der Kulturen wurde durch Spektralphotometrie bei 580 nm verfolgt. Das Ergebnis dieses Versuches ist in Fig. 3 dargestellt. Der Zeitpunkt 0 Minuten bezeichnet hierbei das Anheben der Temperatur von 28 auf 42°C.

## Patentansprüche (Patentansprüche für folgende(n) Vertragsstaat(en): AT, BE, CH, DE, FR, GB, IT, LI, LU, NL, SE)

1. Rekombinante DNA, **dadurch gekennzeichnet,** daß sie für die N-terminale membrandurchdringende Domäne des E-Proteins des Phagen φX 174 kodierende DNA-Sequenzen und für die C-terminale membrandurchdringende Domäne des L-Proteins des Phagen MS2 kodierende DNA-Sequenzen enthält und die DNA-Sequenzen der beiden Phagen durch eine für eine hydrophile flexible Aminosäuresequenz kodierende DNA-Sequenz verbunden sind.

2. Rekombinante DNA nach Anspruch 1,
**dadurch gekennzeichnet,** daß sie die DNA-Sequenz, welche für die Aminosäuren 1 bis 54 des Proteins E des Phagen φX 174 kodiert und die DNA-Sequenz, welche für die Aminosäuren 21 bis 75 des Proteins L des Phagen MS2 kodiert, enthält, wobei diese DNA-Sequenzen durch eine DNA-Sequenz verbunden sind, welche für 5 Linker-Aminosäuren kodiert.

3. Rekombinante DNA nach Anspruch 1 und 2, **dadurch gekennzeichnet,** daß sie eine DNA-Sequenz enthält, welche für eine Aminosäuresequenz, wie sie in Fig. 1 dargestellt ist, kodiert.

4. Rekombinante DNA nach Anspruch 1 bis 3, **dadurch gekennzeichnet,** daß sie eingebaut in einen Vektor vorliegt.

5. Rekombinante DNA nach Anspruch 4, **dadurch gekennzeichnet,** daß der Vektor ein Plasmid oder ein Phagengenom ist.

6. Rekombinante DNA nach Anspruch 1 bis 5, **dadurch gekennzeichnet,** daß sie vor den Phagen-DNA-Sequenzen einen prokaryontischen oder eukaryontischen Promotor, insbesondere einen regulierbaren Promotor enthält, der die Expression steuert.

7. Rekombinante DNA nach Anspruch 6,
**dadurch gekennzeichnet,** daß der Promotor der Lamda-Promotor, der lac-Promotor, oder der Ga110-Promotor ist.

8. Rekombinante DNA nach Anspruch 1 bis 7, **dadurch gekennzeichnet,** daß sie vor den Phagensequenzen zusätzlich eine für eine Signalsequenz kodierende DNA-Sequenz enthält.

9. Plasmid pRM17, DSM 4092P, **dadurch gekennzeichnet,** daß es rekombinante DNA nach Anspruch 3 enthält.

10. Verfahren zur Herstellung einer rekombinanten DNA nach Anspruch 1 bis 3, **dadurch gekennzeichnet,** daß man nach an sich bekannten Methoden die entsprechenden DNA-Sequenzen aus der doppelsträngigen DNA-Form des Phagen φX174 und der DNA-Kopie der Phagen MS2 RNA isoliert und miteinander in der gewünschten Reihenfolge ligiert.

11. Verfahren zur Herstellung rekombinanter DNA nach Anspruch 4 und 5, **dadurch gekennzeichnet,** daß man die nach Anspruch 10 isolierten und ligierten DNA-Sequenzen in einen Vektor insertiert.

12. Verfahren nach Anspruch 11, **dadurch gekennzeichnet,** daß man als Vektor ein Plasmid oder ein Phagengenom verwendet.

13. Verfahren zur Herstellung einer rekombinanten DNA nach Anspruch 6, **dadurch gekennzeichnet,** daß man in den Vektor vor die Phagen-DNA-Sequenz zusätzlich einen eukaryontischen oder prokaryontischen Promotor insertiert.

14. Verfahren nach Anspruch 13, **dadurch gekennzeichnet,** daß man einen Vektor verwendet, der bereits einen Promotor enthält, unter dessen Kontrolle ein Fremdgen exprimiert werden kann.

15. Verfahren zur Herstellung einer rekombinanten DNA nach Anspruch 8, **dadurch gekennzeichnet,** daß man vor die Phagen-DNA-Sequenz zusätzlich eine für eine Signalsequenz kodierende DNA-Sequenz insertiert.

16. Verwendung einer rekombinanten DNA nach Anspruch 1 bis 8 und des Plasmids pRM17, DSM 4092P nach Anspruch 9, zur Gewinnung von eukaryontischen oder prokaryontischen Stoffwechselprodukten oder gentechnologisch hergestellten Proteinen durch partielle oder vollständige Lysis der Zellwand der zur Expression verwendeten Zellen oder Mikroorganismen durch Expression des durch die rekombinante DNA kodierten lytischen Proteins.

17. Verwendung nach Anspruch 16, **dadurch gekennzeichnet,** daß die Expression des lytischen Proteins unter Kontrolle eines regulierbaren Promotors zu einem gewünschten Zeitpunkt induziert wird.

18. Verwendung nach Anspruch 16 und 17,
**dadurch gekennzeichnet,** daß durch das Vorhandensein einer Signalsequenz eine spezifische Lysis bestimmter Membranen erreicht wird.

19. Verwendung nach Anspruch 18, **dadurch gekennzeichnet,** daß die Lysis einer Organellenmembran bewirkt wird.

## Patentansprüche (Patentansprüche für folgende(n) Vertragsstaat(en): ES, GR)

1. Verfahren zur Herstellung einer rekombinanten DNA, die die N-terminale membrandurchdringende Domäne des E-Proteins des Phagen φX 174 kodierende DNA-Sequenzen und für die C-terminale membrandurchdringende Domäne des L-Proteins des Phagen MS2 kodierende DNA-Sequenzen enthält,
**dadurch gekennzeichnet,**
daß man die DNA-Sequenzen der beiden Phagen durch eine für eine hydrophile flexible Aminosäuresequenz kodierende DNA-Sequenz verbindet, indem man nach an sich bekannten Methoden die entsprechenden DNA-Sequenzen aus der doppelsträngigen DNA-Form des Phagen φX174 und der DNA-Kopie der Phagen MS2 RNA isoliert und miteinander in der gewünschten Reihenfolge ligiert.

2. Verfahren nach Anspruch 1,
**dadurch gekennzeichnet,**
daß man die DNA-Sequenz, welche für die Aminosäuren 1 bis 54 des Proteins E des Phagen φX 174 kodiert und die DNA-Sequenz, welche für die Aminosäuren 21 bis 75 des Proteins L des Phagen MS2 kodiert verwendet, und diese DNA-Sequenzen durch eine DNA-Sequenz verbindet, welche für 5 Linker-Aminosäuren kodiert.

3. Verfahren nach Anspruch 1 und 2,
**dadurch gekennzeichnet,**
daß man eine DNA-Sequenz verwendet, welche für eine Aminosäuresequenz, wie sie in Fig. 1 dargestellt ist, kodiert.

4. Verfahren nach Anspruch 1 bis 3,
**dadurch gekennzeichnet,**
daß man die rekombinante DNA in einen Vektor einbaut.

5. Verfahren nach Anspruch 4,
**dadurch gekennzeichnet,**
daß man als Vektor ein Plasmid oder ein Phagengenom verwendet.

6. Verfahren nach Anspruch 1 bis 5,
**dadurch gekennzeichnet,**
daß man vor den Phagen-DNA-Sequenzen einen prokaryontischen oder eukaryontischen Promotor, insbesondere einen regulierbaren Promotor insertiert, der die Expression steuert.

7. Verfahren nach Anspruch 6,
**dadurch gekennzeichnet,**
daß der Promotor der Lamda-Promotor, der lac-Promotor, oder der Gal10-Promotor verwendet wird.

8. Verfahren nach Anspruch 1 bis 7,
**dadurch gekennzeichnet,**
daß man vor den Phagensequenzen zusätzlich eine für eine Signalsequenz kodierende DNA-Sequenz insertiert.

9. Verfahren nach Anspruch 5,
**dadurch gekennzeichnet,**
daß man einen Vektor verwendet, der bereits einen Promotor enthält, unter dessen Kontrolle ein Fremdgen exprimiert werden kann.

10. Verfahren nach Anspruch 1,
**dadurch gekennzeichnet,**
daß man rekombinante DNA nach Anspruch 3 in das Plasmid pSU729 einbaut und Plasmid pRM17, DSM 4092P erhält.

11. Verwendung einer nach Anspruch 1 bis 8 hergestellten rekombinanten DNA und des Plasmids pRM17, DSM 4092P nach Anspruch 10, zur Gewinnung von eukaryontischen oder prokaryontischen Stoffwechselprodukten oder gentechnologisch hergestellten Proteinen durch partielle oder vollständige Lysis der Zellwand der zur Expression verwendeten Zellen oder Mikroorganismen durch Expression des durch die rekombinante DNA kodierten lytischen Proteins.

12. Verwendung nach Anspruch 11,
**dadurch gekennzeichnet,**
daß die Expression des lytischen Proteins unter Kontrolle eines regulierbaren Promotors zu einem gewünschten Zeitpunkt induziert wird.

13. Verwendung nach Anspruch 11 oder 12,
**dadurch gekennzeichnet,**
daß durch das Vorhandensein einer Signalsequenz eine spezifische Lysis bestimmter Membranen erreicht wird.

14. Verwendung nach Anspruch 13,
**dadurch gekennzeichnet,**
daß die Lysis einer Organellenmembran bewirkt wird.

## Claims (Claims for the following Contracting State(s): AT, BE, CH, DE, FR, GB, IT, LI, LU, NL, SE)

1. Recombinant DNA, characterized in that it contains DNA sequences coding for the N-terminal membrane-penetrating domain of the E-protein of the phage φX 174 and DNA sequences coding for the C-terminal membrane-penetrating domain of the L-protein of the phage MS2 and the DNA sequences of both phages are connected by a DNA sequence coding for a hydrophilic flexible amino acid sequence.

2. Recombinant DNA according to claim 1,
characterized in that it contains the DNA sequence which codes for the amino acids 1 to 54 of the protein E of the phage φX 174 and the DNA sequence which codes for the amino acids 21 to 75 of the protein L of the phage MS2, these DNA sequences being connected by a DNA sequence which codes for 5 linker amino acids.

3. Recombinant DNA according to claims 1 and 2, characterized in that it contains a DNA sequence which codes for an amino acid sequence as is illustrated in Fig.1 of the accompanying drawings.

4. Recombinant DNA according to claims 1 to 3, characterized in that it is present incorporated into a vector.

5. Recombinant DNA according to claim 4,
characterized in that the vector is a plasmid or a phage genome.

6. Recombinant DNA according to claims 1 to 5, characterized in that, before the phage DNA sequences, it contains a prokaryotic or eukaryotic promotor, especially a regulatable promotor, which controls the expression.

7. Recombinant DNA according to claim 6,
characterized in that the promotor is the lambda promotor, the lac promotor or the Ga110 promotor.

8. Recombinant DNA according to claims 1 to 7, characterized in that, before the phage sequences, it additionally contains a DNA sequence coding for a signal sequence.

9. Plasmid pRM17, DSM 4092P,
characterized in that it contains recombinant DNA according to claim 3.

10. Process for the production of a recombinant DNA according to claims 1 to 3, characterized in that, according to methods known per se, the corresponding DNA sequences are isolated from the double-stranded DNA form of the phage φX 174 and from the DNA copy of the phage MS2 RNA and ligated with one another in the desired sequence.

11. Process for the production of recombinant DNA according to claim 4 or 5, characterized in that DNA sequences isolated and ligated according to claim 11 are inserted into a vector.

12. Process according to claim 11,
characterized in that a plasmid or phage genome is used as vector.

13. Process for the production of a recombinant DNA according to claim 6, characterized in that, into the vector, before the phage DNA sequence, there is additionally inserted a eukaryotic or prokaryotic promotor.

14. Process according to claim 13,
characterized in that a vector is used which already contains a promotor under the control of which a foreign gene can be expressed.

15. Process for the production of a recombinant DNA according to claim 8, characterized in that, before the phage DNA sequence, there is additionally inserted a DNA sequence coding for a signal sequence.

16. Use of a recombinant DNA according to claims 1 to 8 and of the plasmid pRM17, DSM 4092P, according to claim 9 for obtaining eukaryotic or prokaryotic metabolic products or gene-technologically produced proteins by partial or complete lysis of the cell wall of the cells or micro-organisms used for the expression by expression of the lytic protein coded by the the recombinant DNA.

17. Use according to claim 16,
characterized in that the expression of the lytic protein is induced under the control of a regulatable promotor at a desired point of time.

18. Use according to claim 16 or 17,
characterized in that, due to the presence of a signal sequence, a specific lysis of certain membranes is achieved.

19. Use according to claim 18,
characterized in that the lysis of an organelle membrane is brought about.

## Claims (Claims for the following Contracting State(s): ES, GR)

1. Process for the production of a recombinant DNA which contains the DNA sequences coding for the N-terminal membrane-penetrating domain of the E-protein of the phage φX 174 and the DNA sequences coding for the C-terminal membrane-penetrating domain of the L-protein of the phage MS2, wherein the DNA sequences of both phages are connected by a DNA sequence coding for a hydrophilic flexible amino acid sequence by isolating, according to known methods, the corresponding DNA sequences from the double-stranded DNA form of the phage φX 174 and the DNA copy of the phage MS2 RNA and ligating them with each other in the desired sequence.

2. Process according to claim 1,
wherein the DNA sequence which codes for the amino acids 1 to 54 of the protein E of the phage φX 174 and the DNA sequence which codes for the amino acids 21 to 75 of the protein L of the phage MS2 are used, and these DNA sequences are connected by a DNA sequence which codes for 5 linker amino acids.

3. Process according to claim 1 and 2,
wherein a DNA sequence is used which codes for an amino acid sequence as is illustrated in Fig.1.

4. Process according to claims 1 to 3,
wherein the recombinant DNA is incorporated into a vector.

5. Process according to claim 4,
wherein the vector used is a plasmid or a phage genome.

6. Process according to claims 1 to 5,
wherein a prokaryotic or eukaryotic promotor, in particuar, a regulatable promotor, which controls the expression, is inserted before the phage DNA sequences.

7. Process according to claim 6,
wherein the promotor used is the lambda promotor, the lac promotor or the Ga110 promotor.

8. Process according to claims 1 to 7,
wherein, additionally, a DNA sequence coding for a signal sequence is inserted before the phage sequences.

9. Process according to claim 5,
wherein a vector is used which already contains a promotor under the control of which a foreign gene can be expressed.

10. Process according to claim 1,
wherein recombinant DNA according to claim 3 is incorporated into the plasmid pSU729 and the plasmid pRM17, DSM 4092P, is obtained.

11. Use of a recombinant DNA produced according to any of claims 1 to 8 and of the plasmid pRM17, DSM 4092P, according to claim 10 for obtaining eukaryotic or prokaryotic metabolic products or gene-technologically produced proteins by partial or complete lysis of the cell wall of the cells or microorganisms used for the expression by expression of the lytic protein coded by the recombinant DNA.

12. Use according to claim 11,
wherein the expression of the lytic protein is induced under the control of a regulatable promotor at a desired point of time.

13. Use according to claims 11 or 12,
wherein, due to the presence of a signal sequence, a specific lysis of certain membranes is achieved.

14. Use according to claim 13,
wherein the lysis of an organelle membrane is brought about.

## Revendications (Revendications pour l'(les) Etat(s) contractant(s) suivant(s): AT, BE, CH, DE, FR, GB, IT, LI, LU, NL, SE)

1. ADN recombiné, caractérisé en ce qu'il contient les séquences d'ADN codant pour le domaine transmembranaire N-terminal de la protéine E du phage φX 174 et les séquences d'ADN codant pour le domaine transmembranaire C-terminal de la protéine L du phage MS2, et en ce que les séquences d'ADN des deux phages sont reliées par une séquence ADN codant pour une séquence d'acide aminé flexible hydrophile.

2. ADN recombiné selon la revendication 1, caractérisé en ce qu'il contient la séquence ADN qui code pour les acides aminés 1 à 54 de la protéine E du phage φX 174 et la séquence ADN qui code pour les acides aminés 21 jusqu'à 75 de la protéine L du phage MS2, ces séquences ADN étant reliées par une séquence ADN qui code pour des acides aminés à 5 lieurs.

3. ADN recombiné selon les revendications 1 et 2, caractérisé en ce qu'il contient une séquence d'ADN qui code pour une séquence d'acide aminé telle qu'elle est représentée sur la figure 1.

4. ADN recombiné selon les revendications 1 à 3, caractérisé en ce qu'il se présente incorporé dans un vecteur.

5. ADN recombiné selon la revendication 4, caractérisé en ce que le vecteur est un plasmide ou un génome de phage.

6. ADN recombiné selon les revendications 1 à 5, caractérisé en ce qu'il contient en amont des séquences ADN de phage un promoteur procaryote ou eucaryote, notamment un promoteur régulable qui commande l'expression.

7. ADN recombiné selon la revendication 6, caractérisé en ce que le promoteur est le promoteur Lamda, le promoteur Lac ou le promoteur Gal10.

8. ADN recombiné selon les revendications 1 à 7, caractérisé en ce qu'en amont des séquences de phage, il contient de plus une séquence d'ADN codant pour une séquence de signaux.

9. Plasmide pRM17, DSM 4092P, caractérisé en ce qu'il contient l'ADN recombiné selon la revendication 3.

10. Procédé pour la préparation d'un ADN recombiné selon les revendications 1 à 3, caractérisé en ce que l'on isole, selon des méthodes connues per se, les séquences d'ADN Correspondantes à partir de la forme d'ADN à double brin du phage φX 174 et de la copie d'ADN des phages MS2 ARN et en ce qu'on les ligature ensemble dans l'ordre souhaité.

11. Procédé pour la préparation d'ADN recombiné selon les revendications 4 et 5, caractérisé en ce que l'on insère dans un vecteur les séquences d'ADN isolées et ligaturées selon la revendication 10.

12. Procédé selon la revendication 11, caractérisé en ce qu'en tant que vecteur, on utilise un plasmide ou un génome de phage.

13. Procédé pour la préparation d'un ADN recombiné selon la revendication 6, caractérisé en ce qu'en amont de la séquence ADN de phage, on insère à titre supplémentaire dans le vecteur un promoteur eucaryote ou procaryote.

14. Procédé selon la revendication 13, caractérisé en ce que l'on utilise un vecteur qui contient déjà un promoteur, sous le contrôle duquel on peut exprimer un gène étranger.

15. Procédé pour la préparation d'un ADN recombiné selon la revendication 8, caractérisé en ce qu'en amont de la séquence ADN de phage, on insère une séquence ADN codant pour une séquence de signaux.

16. Utilisation d'un ADN recombiné selon les revendications 1 à 8 et du plasmide pRM17, DSM 4092P selon la revendication 9 pour l'obtention de produits du métabolisme eucaryotes ou procaryotes ou de protéines produites par génie génétique par lyse partielle ou totale de la paroi cellulaire des cellules utilisées pour l'expression ou de micro-organismes par expression de la protéine lytique codée par l'ADN recombiné.

17. Utilisation selon la revendication 16, caractérisée en ce que l'expression de la protéine lytique est induite sous contrôle d'un promoteur régulable à un moment souhaité.

18. Utilisation selon les revendications 16 et 17, caractérisée en ce que l'on parvient à une lyse spécifique de membranes déterminées grâce à l'existence d'une séquence de signaux.

19. Utilisation selon la revendication 18, caractérisée en ce que l'on réalise la lyse d'une membrane organite.

## Revendications (Revendications pour l'(les) Etat(s) contractant(s) suivant(s): ES, GR)

1. Procédé pour la préparation d'un ADN recombiné qui contient les séquence d'ADN codant pour le domaine transmembranaire N-terminal de la protéine E du phage, les séquences d'ADN codant pour le domaine transmembranaire N-terminal de la protéine E du phage φX 174 et les séquences d'ADN codant pour le domaine transmembranaire C-terminal de la protéine L du phage MS2,
caractérisé en ce que l'on relie les séquences d'ADN des deux phages par une séquence d'ADN codant pour une séquence d'acide aminé flexible hydrophile, en isolant selon des méthodes connues les séquences d'ADN correspondantes à partir de la forme d'ADN à double brin du phage φX 174 et de la copie d'ADN des phages MS2 ANR.

2. Procédé selon la revendication 1, caractérisé en ce que l'on utilise la séquence d'ADN qui code pour les acides aminés 1 à 54 de la protéine E du phage φX 174 et la séquence d'ADN qui code pour les acides aminés 21 à 75 de la protéine L du phage MS2, et en ce que l'on relie ces séquences d'ADN par une séquence d'ADN qui code pour les acides aminés à 5 lieurs.

3. Procédé selon les revendications 1 et 2, caractérisé en ce que l'on utilise une séquence d'ADN qui code pour une séquence d'acide aminé telle qu'elle est représentée sur la figure 1.

4. Procédé selon les revendications 1 à 3, caractérisé en ce que l'on incorpore l'ADN recombiné dans un vecteur.

5. Procédé selon la revendication 4, caractérisé en ce que l'on utilise comme vecteur un plasmide ou un génome de phage.

6. Procédé selon les revendications 1 à 5, caractérisé en ce que l'on insère en amont des séquences ADN de phage un promoteur procaryote ou eucaryote, notamment un promoteur régulable qui commande l'expression.

7. Procédé selon la revendication 6, caractérisé en ce que l'on utilise en tant que promoteur le promoteur Lambda, le promoteur Lac ou le promoteur Gal10.

8. Procédé selon les revendications 1 à 7, caractérisé en ce qu'on insère de plus en amont des séquences de phage une séquence d'ADN codant pour une séquence de signaux.

9. Procédé selon la revendication 5, caractérisé en ce que l'on utilise un vecteur qui contient déjà un promoteur sous le contrôle duquel un gène étranger peut être exprimé.

10. Procédé selon la revendication 1, caractérisé en ce que l'on incorpore un ADN recombiné selon la revendication 3 dans le plasmide PSU729 et l'on obtient le plasmide pRM17 DSM 4092P.

11. Utilisation de l'un des ADN recombinés préparés selon les revendications 1 à 8 et du plasmide pRM17 DSM 4092P selon la revendication 10 pour l'obtention de produits du métabolisme eucaryotes ou procaryotes ou de protéines produites par génie génétique par lyse partielle ou totale de la paroi cellulaire de cellules utilisées pour l'expression ou de micro-organismes par expression de la protéine lytique codée par l'ADN recombiné.

12. Utilisation selon la revendication 11, caractérisée en ce que l'expression de la protéine lytique est induite sous contrôle d'un promoteur régulable à un moment souhaité.

13. Utilisation selon la revendication 11 ou 12, caractérisée en ce que l'on parvient à une lyse spécifique de membrane déterminées grâce à l'existence d'une séquence de signaux.

14. Utilisation selon la revendication 13, caractérisée en ce que l'on réalise la lyse d'une membrane organite.
